# EUROPEAN PATENT SPECIFICATION

(11) **EP 4 251 234 B1**
(45) Date of publication and mention of the grant of the patent: **11.03.2026**
(21) Application number: 21824173.5
(22) Date of filing: 15.11.2021
(51) Int. Cl.: A61M 5/14, A61M 39/08

(54) **INTRAVENOUS TUBE UNTANGLING DEVICE**
VORRICHTUNG ZUM ENTWIRREN VON INTRAVENÖSEN SCHLÄUCHEN
DISPOSITIF DE DÉMÊLAGE DE TUBE INTRAVEINEUX

(30) Priority: 24.11.2020 US 202017103476
(43) Date of publication of application: 04.10.2023
(73) Proprietor: CareFusion 303, Inc., San Diego, CA 92130 (US)
(72) Inventor: LAU, Choi Ting, Santee, CA 92701 (US)
(74) Representative: Zenz Patentanwälte Partnerschaft mbB
(86) International application number: PCT/US2021/059402
(87) International publication number: WO 2022/115266

(56) References cited:
- US-A- 3 896 527
- US-A- 4 707 906
- US-A1- 2016 074 628

## Description

### TECHNICAL FIELD

The present disclosure generally relates to a method of operating intravenous (IV) tube organization, in particular IV tube untangling devices.

### BACKGROUND

In typical infusion applications, many IV lines may be used to and from various infusion components, pumps and fluid sources. Due to emergencies or other complications, tangled IV tubing is often inevitable. Tangled IV tubes or lines can create a hazard to both the patient and the health care providers. For example, tangled IV tubes can cause restrictions in fluid flow, restrict movement of attached infusion devices and cause errors in connecting replacement components, pumps or fluid sources. Typical IV tubing organizing solutions require detailed organizing before IV tubes become tangled. However, in a fast pace hospital environment, detailed IV tube organizing may not be possible, in which case IV tube tangling is essentially inevitable.

For these reasons, it is desirable to provide a simple and easy to carry device allowing health care providers to untangle multiple IV tube lines quickly and efficiently, as well as to keep the IV tubes organized after untangling.

US 3 896 527 A discloses a clamp comprising a pair of integral jaws hingedly connected at one end for pivotal movement between opened and closed positions. One jaw contains a latch strike while the other jaw includes a projecting latching member selectively engageable with the latch strike for securely though releasably interconnecting the jaws in various locked positions. The clamp is fabricated from a semi-rigid plastic material having a high fatigue level, and is particularly adapted for releasably securing medical tubing and the like to or immediately adjacent a patient in a medical environment.

US 2016/074628 A1 a catheter clip for managing a medical device having an elongate catheter shaft is provided. The catheter clip comprises a first body section having a c-shape and forming a first opening configured to accommodate a first portion of medical device. The catheter clip further comprises a second body portion having a u-shape and forming a second opening configured to accommodate a plurality of catheter shaft portions of the medical device therein. An inside surface coincident with the second opening includes a least one indentation configured to accommodate the catheter shaft portion therein, and at least one raised portion adjacent to the indentation and configured to inhibit movement of the catheter shaft portion.

US 4 707 906 A discloses a tube holder for hospital tubing comprising an elongated strip with opposing upper and lower faces formed as a plurality of side-by-side, open-ended sleeve like receptacles of varying enclosed cross-sectional area. In one embodiment, the strip is a spring metal piece with a reverse bend forming upper and lower legs with undulating surfaces formed in at least one of the legs, but preferably both of the legs, so that oppositely facing undulations on the upper and lower legs cooperate to form the spaced apart receptacles. Each receptacle has an entrance opening formed by a portion of the upper face being spaced from the lower face by a minimum distance. The entrance opening to each receptacle has a smaller dimension than the maximum dimension of its corresponding receptacle. Each receptacle is movable, say by prying apart the upper and lower legs to increase the spacing at the entrance openings for allowing tubing to slip sideways into any one of the receptacles. A releasable fastener, such as a spring loaded clip, is secured to an end of the strip for holding the tube holder in a fixed position on a bed, sheets, bedclothes, instruments or the like. Hospital tubing of a standard size can be slipped into the larger sized receptacle for confining the tubing but for allowing the tubing to move freely lengthwise through the receptacle. The same tubing can be inserted in the smaller sized receptacle which clamps onto opposite sides of the tubing for securely holding the tubing in place.

### SUMMARY

The present disclosure provides methods configured to receive and/or capture multiple IV tubes and then untangle and straighten the IV tubes by sliding the IV tube untangling device along the IV tubes. The invention as such is defined in the independent claims. Preferable embodiments are further laid out in the dependent claims.

An intravenous (IV) tube untangling device is provided. The IV tube untangling device includes a single-piece integral housing, the housing including a top portion having a first inner surface, a bottom portion having a second inner surface and a flexible interface disposed on a first end of the housing, the flexible interface joining the top portion and the bottom portion. The IV tube untangling device also includes multiple first tubing grooves disposed on the first inner surface and multiple second tubing grooves disposed on the second inner surface, the first and second tubing grooves configured to receive the IV tubes. The flexible interface causes the second inner surface to be at an angle greater than zero degrees in relation to the first inner surface when the housing is in an open position. When the housing is in a closed position, the flexible interface provides a biasing force configured to move the top and bottom portions relative to each other back to the open position.

In one or more examples, the second tubing grooves oppose the first tubing grooves when the housing is in the closed position. In one or more aspects, the opposed first and second tubing grooves are semi-cylindrical shaped. In one or more aspects, the opposed first and second tubing grooves form multiple cylindrical shaped IV tube channels, each channel configured to receive one IV tube. In one or more aspects, multiple guide portions are disposed between the first tubing grooves, each guide portion disposed between a pair of first tubing grooves and configured to guide an IV tube into one of the pair of first tubing grooves. In one or more aspects, multiple guide portions are disposed between the second tubing grooves, each guide portion disposed between a pair of second tubing grooves and configured to guide an IV tube into one of the pair of second tubing grooves.

A securing tab is disposed at a second end of the housing. The securing tab comprises a securing surface that is sized and shaped to engage and hold an outer surface of the top portion when the housing is in the closed position.

The securing tab is configured to flex in a direction away from the first end of the housing when a similarly directed force is applied to the securing tab, and wherein the biasing force of the flexible interface is configured to cause the top portion to move away from the bottom portion when the securing surface moves clear of the outer surface of the top portion. The IV tube untangling device is configured to be slidably moved along the plurality of IV tubes when the housing is in the closed position, causing the IV tubes to one of untangle and straighten.

In one or more examples, an intravenous (IV) tube untangling device is provided. The IV tube untangling device includes a single-piece integral housing, the housing including a top portion having a first inner surface, a bottom portion having a second inner surface, a flexible interface disposed on a first end of the housing, the flexible interface joining the top portion and the bottom portion, and an open second end opposite the first end. The IV tube untangling device also includes multiple first tubing grooves disposed on the first inner surface and multiple second tubing grooves disposed on the second inner surface, the first and second tubing grooves configured to receive the IV tubes. The flexible interface causes the second inner surface to be parallel to the first inner surface when the housing is in closed position. When the housing is in an open position, the flexible interface provides a biasing force configured to move the top and bottom portions relative to each other back to the closed position.

In one or more examples, the second tubing grooves oppose the first tubing grooves when the housing is in the closed position. In one or more aspects, the opposed first and second tubing grooves are semi-cylindrical shaped. In one or more aspects, the opposed first and second tubing grooves form a plurality of cylindrical shaped IV tube channels, each channel configured to receive one IV tube. In one or more aspects, multiple guide portions are disposed between the first tubing grooves, each guide portion disposed between a pair of first tubing grooves and configured to separate the pair of first tubing grooves. In one or more aspects, multiple guide portions are disposed between the second tubing grooves, each guide portion disposed between a pair of second tubing grooves and configured to separate the pair of second tubing grooves.

In one or more examples, when a sufficient opening force is applied to the top and bottom portions to overcome the biasing force of the flexible interface, the housing is configured to cause the top portion to move relative to the bottom portion to form an angle greater than zero degrees between the top and bottom portions. In one or more aspects, the IV tube untangling device is configured to be slidably moved along the plurality of IV tubes when the housing is in the closed position, causing the IV tubes to one of untangle and straighten.

A method of operating an intravenous (IV) tube untangling device is provided. The method includes moving the IV tube untangling device into an open position, moving multiple IV tubes into the IV tube untangling device, guiding each of the IV tubes into one of a groove and a channel of the IV tube untangling device, moving the IV tube untangling device into a closed position, capturing side by side portions of the IV tubes within the IV tube untangling device, slidably moving the IV tube untangling device along the IV tubes, and causing lengthwise portions of the IV tubes to untangle and/or straighten.

In one or more aspects, the method includes moving the IV tube untangling device into an open position again and removing the IV tube untangling device from the IV tubes.

Additional features and advantages of the disclosure will be set forth in the description below and, in part, will be apparent from the description or may be learned by practice of the disclosure. The objectives and other advantages of the disclosure will be realized and attained by the structure particularly pointed out in the written description and claims hereof as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide further understanding of the disclosure and are incorporated in and constitute a part of this specification, illustrate embodiments of the disclosure and together with the description serve to explain the principles of the disclosure.
FIG. 1 is a perspective view of an example patient care system having four fluid infusion pumps, each of which is connected to a respective fluid supply for pumping the contents of the fluid supply to a patient.
FIG. 2 is a perspective view of an example IV tubing tangle.
FIG. 3 is a perspective view of an IV tube untangling device in an open position, according to examples of the disclosure.
Fig. 4 is a front view of the IV tube untangling device of FIG. 3 in a closed position, according to examples of the disclosure.
FIG. 5 is a perspective view of another IV tube untangling device, according to examples of the disclosure.
FIG. 6 is a perspective view of the IV tube untangling device of FIG. 5 with IV tubes, according to examples of the disclosure.
FIG. 7 illustrates a method of operating an IV tube untangling device, according to aspects of the disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

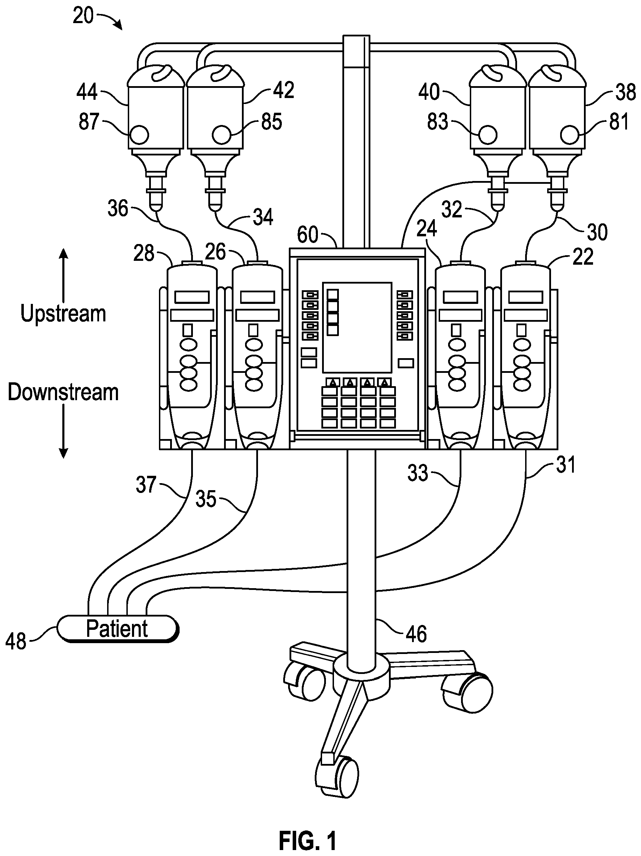

Referring now in more detail to the drawings in which like reference numerals refer to like or corresponding elements among the several views, there is shown in FIG. 1 an example patient care system 20 having four infusion pumps 22, 24, 26, and 28 each of which is fluidly connected with an upstream fluid line 30, 32, 34, and 36, respectively. Each of the four infusion pumps 22, 24, 26, and 28 is also fluidly connected with a downstream fluid line 31, 33, 35, and 37, respectively. The fluid lines can be any type of fluid conduit, such as an IV administration set, through which fluid can flow through. It should be appreciated that any of a variety of pump mechanisms can be used including syringe pumps.

Fluid supplies 38, 40, 42, and 44, which may take various forms but in this case are shown as bottles, are inverted and suspended above the pumps. Fluid supplies may also take the form of bags or other types of containers including syringes. Both the patient care system 20 and the fluid supplies 38, 40, 42, and 44 are mounted to a roller stand, IV pole 46, table top, etc.

A separate infusion pump 22, 24, 26, and 28 is used to infuse each of the fluids of the fluid supplies into the patient. The infusion pumps are flow control devices that will act on the respective fluid line to move the fluid from the fluid supply through the fluid line to the patient 48. Because individual pumps are used, each can be individually set to the pumping or operating parameters required for infusing the particular medical fluid from the respective fluid supply into the patient at the particular rate prescribed for that fluid by the physician. Such medical fluids may include drugs or nutrients or other fluids. The infusion pumps 22, 24, 26, and 28 are controlled by a controller 60.

Fluid supplies 38, 40, 42, and 44 are each coupled to an electronic data tag 81, 83, 85, and 87, respectively, or to an electronic transmitter. Any device or component associated with the infusion system may be equipped with an electronic data tag, reader, or transmitter.

Typically, medical fluid administration sets have more parts than are shown in FIG. 1. Many have check valves, drip chambers, valves with injection ports, connectors, and other devices well known to those skilled in the art, many or all requiring IV tubing. These other devices have not been included in the drawings so as to preserve clarity of illustration.

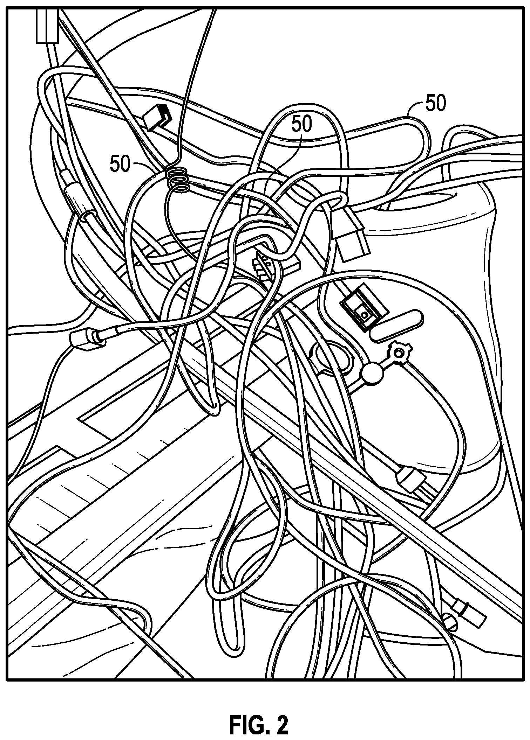

As shown in FIG. 2, a typical infusion set up results in a tangle of IV tubes 50 at the bedside. As discussed above, this tangle may happen as a result of fast paced emergency actions and/or simply due to the fast pace required of care givers in an infusion setting. As seen, it may quickly become very difficult to determine which IV tube 50 is connected to which infusion device, component or fluid source. Further, the tangle of IV tubes 50 can cause any given IV tube 50 to kink, thus restricting fluid flow through that IV tube 50. In addition, the tangle of IV tubes 50 can also restrict movement of any given IV tube 50, which correspondingly restricts movement of the attached infusion device, such as an IV pole with a fluid bag, for example.

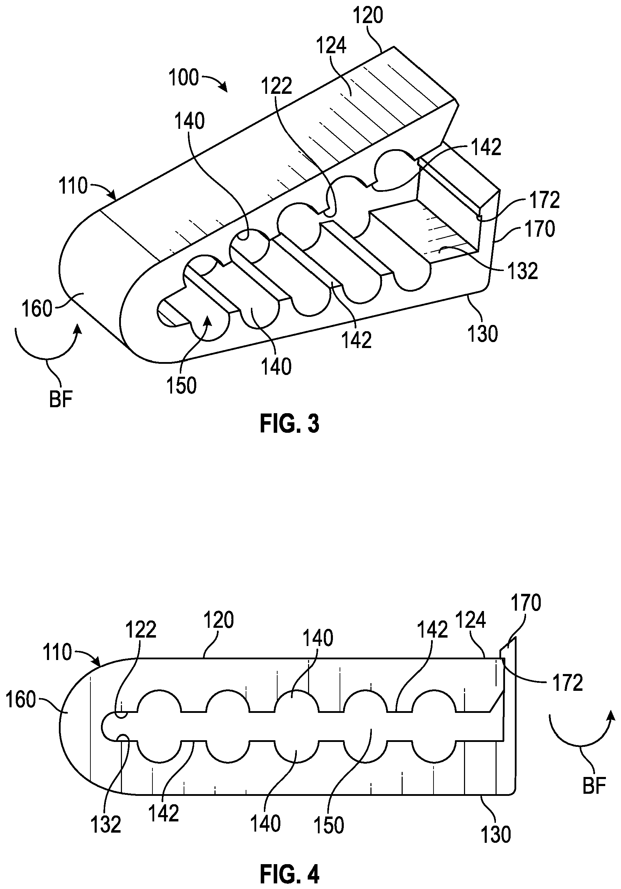

With reference to FIGS. 3 and 4, an IV tube untangling device 100 is shown. The IV tube untangling device 100 may be a one piece housing 110 having a top portion 120 with a top inner surface 122 and a bottom portion 130 with a bottom inner surface 132. Multiple tubing grooves 140 are disposed on each of the top and bottom inner surfaces 122, 132. The tubing grooves 140 on the top portion 120 may directly oppose the tubing grooves 140 on the bottom portion 130. The tubing grooves 140 may be semi-cylindrically shaped, such that when the IV tube untangling device 100 is in a closed and/or secured position the opposing tubing grooves 140 form channels 150 each having a cylindrical shape essentially mirroring the shape of an IV tube 50. Tubing grooves 140 may be any suitable shape, such as oval, rectangular, square or triangular, for example. Guide portions 142 are disposed between the tubing grooves 140.

A flexible interface 160 may be disposed on one end of the housing 110, the flexible interface 160 joining the top portion 120 to the bottom 130 portion with a biasing force BF (e.g., spring like tension). The biasing force BF may bias the IV tube untangling device 100 towards an open position where the top portion 120 is at an angle in relation to the bottom portion 130. A securing tab 170 may be disposed on the bottom portion 130 at the opposite end of the housing 110 from the flexible interface 160. The securing tab 170 includes a securing surface 172 that is sized and shaped to engage and hold an outer surface 124 of the top portion 120.

In use, the top portion 120 may be pushed down or towards the bottom portion 130 so that the securing tab 170 engages the top portion 120, then the top portion 120 continues to be pushed toward the bottom portion 130 until the securing surface 172 engages and holds the outer surface 124 of the top portion 120. The biasing force BF of the flexible interface 160 will cause the top portion 120 to move away from the bottom portion 130 (e.g., pop open) when the securing tab 170 is moved (e.g., pulled) outward to free the outer surface 124 from contact with the securing surface 172. Portions of the top portion 120 and the securing tab 170 may be chamfered or angled to facilitate slidable movement between the top portion 120 and the securing tab 170.

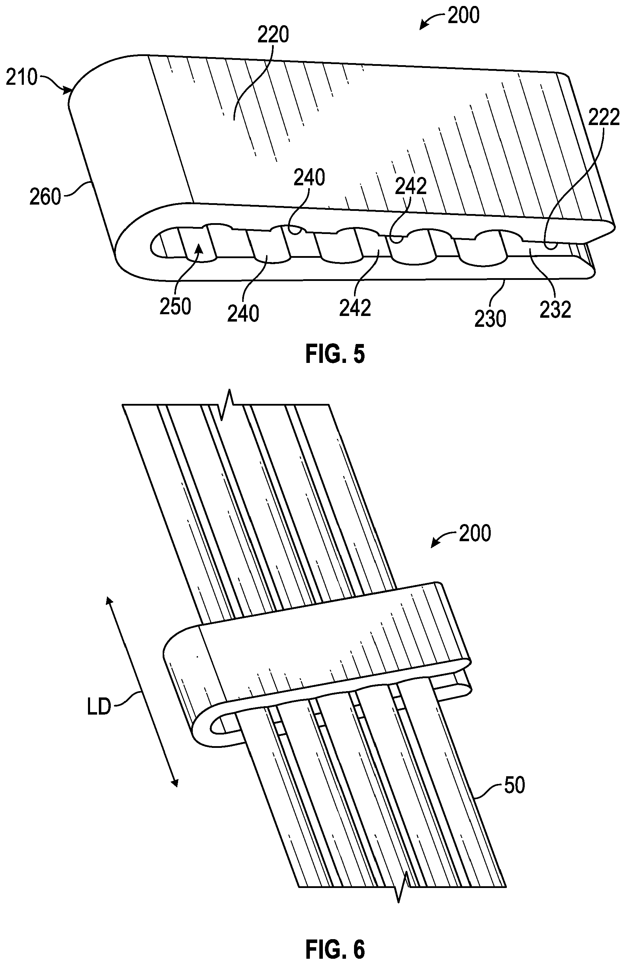

When the IV tubes 50 are engaged and/or secured within the channels 150, the IV tube untangling device 100 may be slidably moved along the lengths of the IV tubes 50 to straighten out tangles between the IV tubes 50 (see FIG. 6). After straightening out the tangled IV tubes 50, the IV tube untangling device 100 may be opened and removed from the straightened IV tubes 50, where the IV tube untangling device 100 may then be used again at another tangle of IV tubes 50. According to aspects of the disclosure, the IV tube untangling device 100 may be left in place after untangling the IV tubes 50, thereby providing a continuous organization of the engaged IV tubes 50.

With reference to FIGS. 5 and 6, an IV tube untangling device 200 is shown. The IV tube untangling device 200 may be a one piece housing 210 having a top portion 220 with a top inner surface 222 and a bottom portion 230 with a bottom inner surface 232. Multiple tubing grooves 240 are disposed on each of the top and bottom inner surfaces 222, 232. The tubing grooves 240 on the top portion 220 may directly oppose the tubing grooves 240 on the bottom portion 230. The tubing grooves 240 may be semi-cylindrically shaped, such that when the IV tube untangling device 200 is in a closed 2 position the opposing tubing grooves 240 form channels 250 each having a cylindrical shape essentially mirroring the shape of an IV tube 50. Tubing grooves 240 may be any suitable shape, such as oval, rectangular, square or triangular, for example. Guide portions 242 are disposed between the tubing grooves 240.

A flexible interface 260 may be disposed on one end of the housing 210, the flexible interface 260 joining the top portion 220 to the bottom 230 portion with a biased or spring like tension. The tension may bias the IV tube untangling device 200 towards a closed position where the top portion 220 is essentially parallel to the bottom portion 230. Unlike the IV tube untangling device 100, IV tube untangling device 200 may not have a securing tab, but instead relies on the biasing force of the flexible interface 260 to remain in the closed position.

In use, the top portion 220 may be pulled up or away from the bottom portion 230 to overcome the bias force and to allow IV tubes 50 to be moved into the tubing grooves 240.

Once the IV tubes 50 are guided into the tubing grooves 240 (e.g., the tubing grooves 240 of the bottom portion 230), the pulling force exerted on the top and/or bottom portions 220, 230 may cease (e.g., user lets go), and the bias force of the flexible interface 260 causes the top portion 220 to move toward the bottom portion 230 (e.g., snap closed) and engage and/or secure the IV tubes 50 in the channels 250.

When the IV tubes 50 are engaged and/or secured within the channels 250, the IV tube untangling device 200 may be slidably moved in either lengthwise direction LD along the lengths of the IV tubes 50 to straighten out tangles between the IV tubes 50. After straightening out the tangled IV tubes 50, the IV tube untangling device 200 may be opened and removed from the straightened IV tubes 50, where the IV tube untangling device 200 may then be used again at another tangle of IV tubes 50. According to aspects of the disclosure, the IV tube untangling device 200 may be left in place after untangling the IV tubes 50, thereby providing a continuous organization of the engaged IV tubes 50.

The IV tube untangling device 100, 200 is a simple and user-friendly device that may be manufactured at a low cost and easily carried around by health care providers, such as in their pockets. The one-piece configuration of the IV tube untangling device 100, 200 easily allows users to quickly capture and untangle multiple IV tubes 50 in fast paced medical environments. The IV tube untangling device 100, 200 is not required to be mounted or connected to any other devices, thus allowing the user to easily carry the IV tube untangling device 100, 200 around for immediate use at any time.

The IV tube untangling device 100, 200 may be used during manufacture and assembly of IV sets. For example, the IV tube untangling device 100, 200 may be packaged together with the IV set as a tubing organizer in place of tape during the distribution and storage of the IV set. Once the IV set is removed from the packaging for use, the IV tube untangling device 100, 200 may then be carried around and/or used as discussed above.

IV tube untangling device 100, 200 may be made from any suitable material for infusion uses, such as plastic or metal, for example. The biasing force of the flexible interface 160, 260 may vary based on the type of material used.

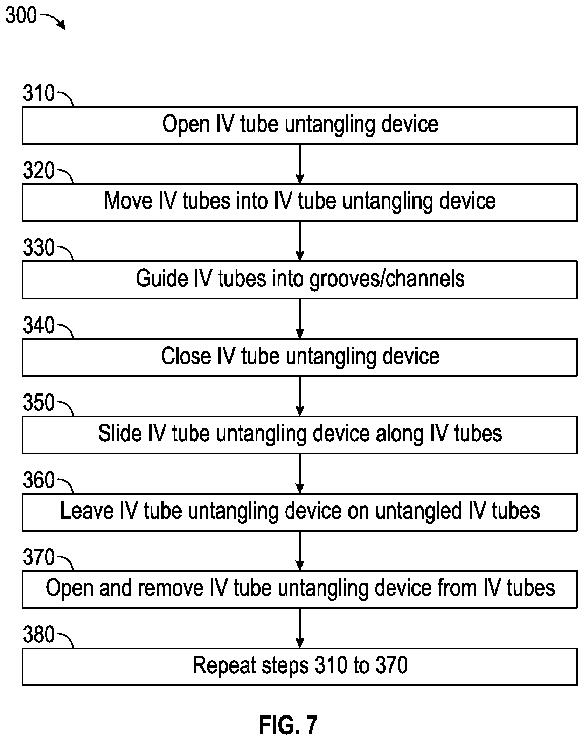

According to some aspects of the disclosure, a method 300 of operating an IV tube untangling device (e.g., IV tube untangling device 100, 200) is shown in FIG. 7. In step 310, the IV tube untangling device is moved (e.g., biased, pulled) into an open position. Multiple IV tubes (e.g., IV tubes 50) are moved into the IV tube untangling device in step 320. Here, the IV tube untangling device may be moved onto a set of IV tubes or the IV tubes may be placed into the IV tube untangling device. In step 330, the IV tubes are guided into grooves (e.g., tubing grooves 140, 240) and/or channels (e.g., channels 150, 250) of the IV tube untangling device. Here, guiding the IV tubes may be done by guides (e.g., guide portions 142, 242) in the IV tube untangling device.

The IV tube untangling device is secured in a closed position in step 340, slidably capturing the IV tubes within the IV tube untangling device. For example, the IV tube untangling device may be forced closed to overcome a biasing force trying to keep the IV tube untangling device open. Here a locking tab (e.g., securing tab 170) may keep the IV tube untangling device in the closed position, thus preventing the biasing force from opening the IV tube untangling device. As another example, a force overcoming a biasing force trying to keep the IV tube untangling device closed may be released, causing the IV tube untangling device to snap closed due to the biasing force towards the closed position. Here, the IV tubes will remain captured in the IV tube untangling device due to the biasing force in the closed direction.

In step 350, the IV tube untangling device is moved along the IV tubes to untangle and/or straighten the IV tubes. The IV tube untangling device is left in a closed position on the untangled/straightened IV tubes to provide continued organization of the IV tubes in step 360. In step 370, the IV tube untangling device is opened and removed from the untangled/straightened IV tubes. The IV tube untangling device is used again in step 380 by repeating any or all of the steps of 310 to 370.

In one or more embodiments, an intravenous (IV) tube untangling device comprises a single-piece integral housing. The single-piece integral housing comprises a top portion having a first inner surface; a bottom portion having a second inner surface; and a flexible interface disposed on a first end of the housing, the flexible interface joining the top portion and the bottom portion. The IV tube untangling device also comprises a plurality of first tubing grooves disposed on the first inner surface; and a plurality of second tubing grooves disposed on the second inner surface, the first and second tubing grooves configured to receive a plurality of IV tubes. The flexible interface causes the second inner surface to be at an angle greater than zero degrees in relation to the first inner surface when the housing is in an open position. Also, when the housing is in a closed position, the flexible interface provides a biasing force configured to move the top and bottom portions relative to each other back to the open position.

In aspects of the disclosure, the second tubing grooves oppose the first tubing grooves when the housing is in the closed position. In aspects of the disclosure, the opposed first and second tubing grooves are semi-cylindrical shaped. In aspects of the disclosure, the opposed first and second tubing grooves form a plurality of cylindrical shaped IV tube channels, each channel configured to receive one IV tube. In aspects of the disclosure, a plurality of guide portions is disposed between the first tubing grooves, each guide portion disposed between a pair of first tubing grooves and configured to guide an IV tube into one of the pair of first tubing grooves. In aspects of the disclosure, a plurality of guide portions is disposed between the second tubing grooves, each guide portion disposed between a pair of second tubing grooves and configured to guide an IV tube into one of the pair of second tubing grooves.

In aspects of the disclosure, a securing tab is disposed at a second end of the housing. In aspects of the disclosure, the securing tab comprises a securing surface that is sized and shaped to engage and hold an outer surface of the top portion when the housing is in the closed position. In aspects of the disclosure, the securing tab is configured to flex in a direction away from the first end of the housing when a similarly directed force is applied to the securing tab, and wherein the biasing force of the flexible interface is configured to cause the top portion to move away from the bottom portion when the securing surface moves clear of the outer surface of the top portion. In aspects of the disclosure, the IV tube untangling device is configured to be slidably moved along the plurality of IV tubes when the housing is in the closed position, causing the IV tubes to one of untangle and straighten.

In one or more embodiments, an intravenous (IV) tube untangling device comprises a single-piece integral housing. The single-piece integral housing comprises a top portion having a first inner surface; a bottom portion having a second inner surface; a flexible interface disposed on a first end of the housing, the flexible interface joining the top portion and the bottom portion; and an open second end opposite the first end. The IV tube untangling device also comprises a plurality of first tubing grooves disposed on the first inner surface; and a plurality of second tubing grooves disposed on the second inner surface, the first and second tubing grooves configured to receive a plurality of IV tubes. The flexible interface causes the second inner surface to be parallel to the first inner surface when the housing is in closed position. Also, when the housing is in an open position, the flexible interface provides a biasing force configured to move the top and bottom portions relative to each other back to the closed position.

In aspects of the disclosure, the second tubing grooves oppose the first tubing grooves when the housing is in the closed position. In aspects of the disclosure, the opposed first and second tubing grooves are semi-cylindrical shaped. In aspects of the disclosure, the opposed first and second tubing grooves form a plurality of cylindrical shaped IV tube channels, each channel configured to receive one IV tube. In aspects of the disclosure, a plurality of guide portions is disposed between the first tubing grooves, each guide portion disposed between a pair of first tubing grooves and configured to separate the pair of first tubing grooves. In aspects of the disclosure, a plurality of guide portions is disposed between the second tubing grooves, each guide portion disposed between a pair of second tubing grooves and configured to separate the pair of second tubing grooves. In aspects of the disclosure, when a sufficient opening force is applied to the top and bottom portions to overcome the biasing force of the flexible interface, the housing is configured to cause the top portion to move relative to the bottom portion to form an angle greater than zero degrees between the top and bottom portions. In aspects of the disclosure, the IV tube untangling device is configured to be slidably moved along the plurality of IV tubes when the housing is in the closed position, causing the IV tubes to one of untangle and straighten.

In one or more embodiments, a method of operating an intravenous (IV) tube untangling device comprises moving the IV tube untangling device into an open position; moving a plurality of IV tubes into the IV tube untangling device; guiding each of the plurality of IV tubes into one of a groove and a channel of the IV tube untangling device; moving the IV tube untangling device into a closed position, capturing side by side portions of the plurality of IV tubes within the IV tube untangling device; slidably moving the IV tube untangling device along the plurality of IV tubes; and causing lengthwise portions of the plurality of IV tubes to one of untangle and straighten.

In aspects of the disclosure, the method comprises moving the IV tube untangling device into an open position again; and removing the IV tube untangling device from the plurality of IV tubes.

As used herein, the terms "control" or "controlling" encompass a wide variety of actions. For example, "controlling" a device may include transmitting one or more messages to adjust an operational state or functional element of the device. The message may include specific instructions to be executed by a processor of the device to manifest the change. The "controlling" may include storing a value in a location of a storage device for subsequent retrieval by the device to be controlled, transmitting a value directly to the device to be controlled via at least one wired or wireless communication medium, transmitting or storing a reference to a value, and the like. For example, a control message may include a value to adjust a level of power from a power source of the controlled device. As another example, a control message may activate or deactivate a structural element of the controlled device such as a light, audio playback, a motor, a lock, a pump, a display, or other component of a device described herein. "Controlling" may include indirect control of the device by adjusting a configuration value used by the controlled device. For example, the control message may include a threshold value for a device characteristic (e.g., temperature, rate, frequency, etc.). The threshold value may be stored in a memory location and referred to by the controlled device during operation.

According to some aspects of the disclosure, a pump assembly includes a fluid flow pump, a tubing pathway configured to receive a fluid tube and a tubing dimension measurement assembly. The tubing dimension measurement assembly includes a processor, an emitter spaced from the tubing pathway and configured to generate an emission into the tubing pathway, and a collector spaced from the tubing pathway, the collector disposed to receive the emission from the emitter, wherein the tubing dimension measurement assembly is further configured to measure an outer diameter (OD) of a tube received in the pathway, wherein said measurement is based at least in part on the emission.

It is understood that any specific order or hierarchy of blocks in the methods of processes disclosed is an illustration of example approaches. Based upon design or implementation preferences, it is understood that the specific order or hierarchy of blocks in the processes may be rearranged, or that all illustrated blocks be performed. In some implementations, any of the blocks may be performed simultaneously.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like.

"Providing" may also include encoding, decoding, encrypting, decrypting, validating, verifying, inserting and the like via a hardware element.

As used herein, the term "message" encompasses a wide variety of formats for communicating (e.g., transmitting or receiving) information. A message may include a machine readable aggregation of information such as an XML document, fixed field message, comma separated message, or the like. A message may, in some implementations, include a signal utilized to transmit one or more representations of the information. While recited in the singular, it will be understood that a message may be composed, transmitted, stored, received, etc. in multiple parts.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, operations or processes disclosed is an illustration of exemplary approaches. Based upon design preferences, it is understood that the specific order or hierarchy of steps, operations or processes may be rearranged. Some of the steps, operations or processes may be performed simultaneously. Some or all of the steps, operations, or processes may be performed automatically, without the intervention of a user. The accompanying method claims, if any, present elements of the various steps, operations or processes in a sample order, and are not meant to be limited to the specific order or hierarchy presented.

Moreover, nothing disclosed herein is intended to be dedicated to the public regardless of whether such disclosure is explicitly recited in the claims. Furthermore, to the extent that the term "include," "have," or the like is used, such term is intended to be inclusive in a manner similar to the term "comprise" as "comprise" is interpreted when employed as a transitional word in a claim.

In any embodiment, data can be forwarded to a "remote" device or location," where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

Some embodiments include implementation on a single computer, or across a network of computers, or across networks of networks of computers, for example, across a network cloud, across a local area network, on hand-held computer devices, etc. The computers may be physical machines or virtual machines hosted by other computers. In certain embodiments, one or more of the steps described herein are implemented on a computer program(s). Such computer programs execute one or more of the steps described herein. In some embodiments, implementations of the subject method include various data structures, categories, and modifiers described herein, encoded on computer-readable medium(s) and transmissible over communications network(s).

Software, web, internet, cloud, or other storage and computer network implementations of the present invention could be accomplished with standardized programming techniques specifically adapted to cause one or more device to perform the various assigning, calculating, identifying, scoring, accessing, generating or discarding steps described.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

## Claims

1. A method of operating an intravenous IV tube untangling device (100, 200), comprising a single-piece integral housing (110, 210) comprising:
a top portion (120, 220) having a first inner surface (122, 222);
a bottom portion (130, 230) having a second inner surface (132, 232);
a flexible interface (160, 260) disposed on a first end of the housing (110, 210), the flexible interface (160, 260) joining the top portion (120, 220) and the bottom portion (130, 230); and
a securing tab disposed at a second end of the housing (110, 210);
a plurality of first tubing grooves (140, 240) disposed on the first inner surface (122, 222); and
a plurality of second tubing grooves (140, 240) disposed on the second inner surface (132, 232), the first and second tubing grooves (140, 240) configured to receive a plurality of IV tubes (50), the method comprising:
moving the IV tube untangling device (100, 200) into an open position;
moving a plurality of IV tubes (50) into the IV tube untangling device (100, 200);
guiding each of the plurality of IV tubes (50) into one of a groove (140, 240) and a channel (150, 250) of the IV tube untangling device (100, 200);
moving the IV tube untangling device (100, 200) into a closed position, capturing side by side portions of the plurality of IV tubes (50) within the IV tube untangling device (100, 200);
slidably moving the IV tube untangling device (100, 200) along the plurality of IV tubes (50); and
causing lengthwise portions of the plurality of IV tubes (50) to one of untangle and straighten,
**characterized by** wherein the securing tab is configured to flex in a direction away from the first end of the housing (110, 210) when a similarly directed force is applied to the securing tab, wherein the securing tab comprises a securing surface that is sized and shaped to engage and hold an outer surface of the top portion (120, 220) when the housing (110, 210) is in the closed position and wherein a biasing force of the flexible interface (160, 260) is configured to cause the top portion (120, 220) to move away from the bottom portion (130, 230) when the securing surface moves clear of the outer surface of the top portion (120, 220).

2. The method of claim 1, further comprising:
moving the IV tube untangling device (100, 200) into an open position again; and
removing the IV tube untangling device (100, 200) from the plurality of IV tubes (50).

3. The method of claim 1,
wherein the flexible interface (160, 260) causes the second inner surface (132, 232) to be at an angle greater than zero degrees in relation to the first inner surface (122, 222) when the housing (110, 210) is in an open position,
and wherein when the housing (110, 210) is in a closed position, the flexible interface (160, 260) provides a biasing force (BF) configured to move the top and bottom portions (120, 220), (130, 230) relative to each other back to the open position.

4. The method of any of claims 1 to 3, wherein the second tubing grooves (140, 240) oppose the first tubing grooves (140, 240) when the housing (110, 210) is in the closed position.

5. The method of claim 4, wherein the opposed first and second tubing grooves (140, 240) are semi-cylindrical shaped.

6. The method of claim 5, wherein the opposed first and second tubing grooves (140, 240) form a plurality of cylindrical shaped IV tube channels (150, 250), each channel (150, 250) configured to receive one IV tube (50).

7. The method of any of claims 1 to 3, wherein a plurality of guide portions (142, 242) are disposed between the first tubing grooves (140, 240), each guide portion (142, 242) disposed between a pair of first tubing grooves (140, 240) and configured to guide an IV tube (50) into one of the pair of first tubing grooves (140, 240).

8. The method of any of claims 1 to 3, wherein a plurality of guide portions (142, 242) are disposed between the second tubing grooves (140, 240), each guide portion (142, 242) disposed between a pair of second tubing grooves (140, 240) and configured to guide an IV tube (50) into one of the pair of second tubing grooves (140, 240).

9. The method of any of claims 1 to 3, wherein the IV tube untangling device is configured to be slidably moved along the plurality of IV tubes (50) when the housing (110, 210) is in the closed position, causing the IV tubes (50) to one of untangle and straighten.

## Patentansprüche

1. Verfahren zum Betreiben einer IV-Schlauch-Entwirrvorrichtung (100, 200), umfassend ein einstückiges integrales Gehäuse (110, 210), umfassend:
einen oberen Abschnitt (120, 220) mit einer ersten Innenfläche (122, 222);
einen unteren Abschnitt (130, 230) mit einer zweiten Innenfläche (132, 232);
eine flexible Schnittstelle (160, 260), die an einem ersten Ende des Gehäuses (110, 210) angeordnet ist, wobei die flexible Schnittstelle (160, 260) den oberen Abschnitt (120, 220) und den unteren Abschnitt (130, 230) verbindet; und
eine Sicherungslasche, die an einem zweiten Ende des Gehäuses (110, 210) angeordnet ist;
eine Mehrzahl erster Schlauchnuten (140, 240), die auf der ersten Innenfläche (122, 222) angeordnet sind; und
eine Mehrzahl zweiter Schlauchnuten (140, 240), die auf der zweiten Innenfläche (132, 232) angeordnet sind, wobei die ersten und zweiten Schlauchnuten (140, 240) dazu konfiguriert sind, eine Mehrzahl von IV-Schläuchen (50) aufzunehmen, wobei das Verfahren umfasst:
Bewegen der IV-Schlauch-Entwirrvorrichtung (100, 200) in eine offene Position;
Bewegen einer Mehrzahl von IV-Schläuchen (50) in die IV-Schlauch-Entwirrvorrichtung (100, 200);
Führen jedes der Mehrzahl von IV-Schläuchen (50) in eine Nut (140, 240) oder einen Kanal (150, 250) der IV-Schlauch-Entwirrvorrichtung (100, 200);
Bewegen der IV-Schlauch-Entwirrvorrichtung (100, 200) in eine geschlossene Position, wobei nebeneinanderliegende Abschnitte der Mehrzahl von IV-Schläuchen (50) innerhalb der IV-Schlauch-Entwirrvorrichtung (100, 200) erfasst werden;
schiebbares Bewegen der IV-Schlauch-Entwirrvorrichtung (100, 200) entlang der Mehrzahl von IV-Schläuchen (50); und
Bewirken, dass längsgerichtete Abschnitte der Mehrzahl von IV-Schläuchen (50) entwirrt oder begradigt werden,
**dadurch gekennzeichnet, dass** die Sicherungslasche dazu konfiguriert ist, sich in eine Richtung weg von dem ersten Ende des Gehäuses (110, 210) zu biegen, wenn eine gleichgerichtete Kraft auf die Sicherungslasche aufgebracht wird, wobei die Sicherungslasche eine Sicherungsfläche umfasst, die derart bemessen und geformt ist, dass sie eine Außenfläche des oberen Abschnitts (120, 220) eingreift und hält, wenn das Gehäuse (110, 210) in der geschlossenen Position ist, und wobei eine Vorspannkraft der flexiblen Schnittstelle (160, 260) dazu konfiguriert ist, zu bewirken, dass sich der obere Abschnitt (120, 220) von dem unteren Abschnitt (130, 230) weg bewegt, wenn sich die Sicherungsfläche von der Außenfläche des oberen Abschnitts (120, 220) löst.

2. Verfahren nach Anspruch 1, ferner umfassend:
erneutes Bewegen der IV-Schlauch-Entwirrvorrichtung (100, 200) in eine offene Position; und
Entfernen der IV-Schlauch-Entwirrvorrichtung (100, 200) von der Mehrzahl von IV-Schläuchen (50).

3. Verfahren nach Anspruch 1, wobei die flexible Schnittstelle (160, 260) bewirkt, dass die zweite Innenfläche (132, 232) bei in einer offenen Position befindlichem Gehäuse (110, 210) in einem Winkel größer als null Grad relativ zu der ersten Innenfläche (122, 222) steht,
und wobei die flexible Schnittstelle (160, 260) bei in einer geschlossenen Position befindlichem Gehäuse (110, 210) eine Vorspannkraft (BF) bereitstellt, die dazu konfiguriert ist, den oberen und den unteren Abschnitt (120, 220), (130, 230) relativ zueinander zurück in die offene Position zu bewegen.

4. Verfahren nach einem der Ansprüche 1 bis 3, wobei die zweiten Schlauchnuten (140, 240) den ersten Schlauchnuten (140, 240) gegenüberliegen, wenn das Gehäuse (110, 210) in der geschlossenen Position ist.

5. Verfahren nach Anspruch 4, wobei die gegenüberliegenden ersten und zweiten Schlauchnuten (140, 240) halbzylindrisch geformt sind.

6. Verfahren nach Anspruch 5, wobei die gegenüberliegenden ersten und zweiten Schlauchnuten (140, 240) eine Mehrzahl zylindrisch geformter IV-Schlauchkanäle (150, 250) bilden, wobei jeder Kanal (150, 250) dazu konfiguriert ist, einen IV-Schlauch (50) aufzunehmen.

7. Verfahren nach einem der Ansprüche 1 bis 3, wobei eine Mehrzahl von Führungsabschnitten (142, 242) zwischen den ersten Schlauchnuten (140, 240) angeordnet ist, wobei jeder Führungsabschnitt (142, 242) zwischen einem Paar erster Schlauchnuten (140, 240) angeordnet ist und dazu konfiguriert ist, einen IV-Schlauch (50) in eine der ersten Schlauchnuten (140, 240) des Paars zu führen.

8. Verfahren nach einem der Ansprüche 1 bis 3, wobei eine Mehrzahl von Führungsabschnitten (142, 242) zwischen den zweiten Schlauchnuten (140, 240) angeordnet ist, wobei jeder Führungsabschnitt (142, 242) zwischen einem Paar zweiter Schlauchnuten (140, 240) angeordnet ist und dazu konfiguriert ist, einen IV-Schlauch (50) in eine der zweiten Schlauchnuten (140, 240) des Paars zu führen.

9. Verfahren nach einem der Ansprüche 1 bis 3, wobei die IV-Schlauch-Entwirrvorrichtung dazu konfiguriert ist, bei in der geschlossenen Position befindlichem Gehäuse (110, 210) schiebbar entlang der Mehrzahl von IV-Schläuchen (50) bewegt zu werden, wodurch bewirkt wird, dass die IV-Schläuche (50) entwirrt oder begradigt werden.

## Revendications

1. Procédé de fonctionnement d'un dispositif de démêlage de tube IV intraveineux (100, 200), comprenant un boîtier (110, 210) intégral monobloc comprenant :
une partie supérieure (120, 220) ayant une première surface interne (122, 222) ;
une partie inférieure (130, 230) ayant une deuxième surface interne (132, 232) ;
une interface flexible (160, 260) disposée à une première extrémité du boîtier (110, 210), l'interface flexible (160, 260) joignant la partie supérieure (120, 220) et la partie inférieure (130, 230) ; et
une languette de fixation disposée à une deuxième extrémité du boîtier (110, 210) ;
une pluralité de premières rainures de tube (140, 240) disposées sur la première surface interne (122, 222) ; et
une pluralité de deuxièmes rainures de tube (140, 240) disposées sur la deuxième surface interne (132, 232), les premières et deuxièmes rainures de tube (140, 240) étant configurées pour recevoir une pluralité de tubes IV (50), le procédé comprenant :
déplacer le dispositif de démêlage de tube IV (100, 200) vers une position ouverte ;
déplacer une pluralité de tubes IV (50) dans le dispositif de démêlage de tube IV (100, 200) ;
guider chacun de la pluralité de tubes IV (50) dans l'une d'une rainure (140, 240) et d'un canal (150, 250) du dispositif de démêlage de tube IV (100, 200) ;
déplacer le dispositif de démêlage de tube IV (100, 200) vers une position fermée, en capturant des portions côte à côte de la pluralité de tubes IV (50) à l'intérieur du dispositif de démêlage de tube IV (100, 200) ;
déplacer de manière coulissante le dispositif de démêlage de tube IV (100, 200) le long de la pluralité de tubes IV (50) ; et
amener des portions longitudinales de la pluralité de tubes IV (50) à être démêlées ou redressées,
**caractérisé en ce que** la languette de fixation est configurée pour fléchir dans une direction s'éloignant de la première extrémité du boîtier (110, 210) lorsqu'une force orientée de manière similaire est appliquée à la languette de fixation, la languette de fixation comprenant une surface de fixation dimensionnée et formée pour s'engager sur et retenir une surface externe de la partie supérieure (120, 220) lorsque le boîtier (110, 210) est en position fermée, et **en ce qu'**une force de rappel de l'interface flexible (160, 260) est configurée pour amener la partie supérieure (120, 220) à s'éloigner de la partie inférieure (130, 230) lorsque la surface de fixation se dégage de la surface externe de la partie supérieure (120, 220).

2. Procédé selon la revendication 1, comprenant en outre :
déplacer à nouveau le dispositif de démêlage de tube IV (100, 200) vers une position ouverte ; et
retirer le dispositif de démêlage de tube IV (100, 200) de la pluralité de tubes IV (50).

3. Procédé selon la revendication 1,
dans lequel l'interface flexible (160, 260) amène la deuxième surface interne (132, 232) à être à un angle supérieur à zéro degré par rapport à la première surface interne (122, 222) lorsque le boîtier (110, 210) est en position ouverte,
et dans lequel, lorsque le boîtier (110, 210) est en position fermée, l'interface flexible (160, 260) fournit une force de rappel (BF) configurée pour déplacer la partie supérieure et la partie inférieure (120, 220), (130, 230) l'une par rapport à l'autre vers la position ouverte.

4. Procédé selon l'une quelconque des revendications 1 à 3, dans lequel les deuxièmes rainures de tube (140, 240) s'opposent aux premières rainures de tube (140, 240) lorsque le boîtier (110, 210) est en position fermée.

5. Procédé selon la revendication 4, dans lequel les premières et deuxièmes rainures de tube opposées (140, 240) sont de forme semi-cylindrique.

6. Procédé selon la revendication 5, dans lequel les premières et deuxièmes rainures de tube opposées (140, 240) forment une pluralité de canaux de tube IV (150, 250) de forme cylindrique, chaque canal (150, 250) étant configuré pour recevoir un tube IV (50).

7. Procédé selon l'une quelconque des revendications 1 à 3, dans lequel une pluralité de portions de guidage (142, 242) sont disposées entre les premières rainures de tube (140, 240), chaque portion de guidage (142, 242) étant disposée entre une paire de premières rainures de tube (140, 240) et étant configurée pour guider un tube IV (50) dans l'une des premières rainures de tube (140, 240) de la paire.

8. Procédé selon l'une quelconque des revendications 1 à 3, dans lequel une pluralité de portions de guidage (142, 242) sont disposées entre les deuxièmes rainures de tube (140, 240), chaque portion de guidage (142, 242) étant disposée entre une paire de deuxièmes rainures de tube (140, 240) et étant configurée pour guider un tube IV (50) dans l'une des deuxièmes rainures de tube (140, 240) de la paire.

9. Procédé selon l'une quelconque des revendications 1 à 3, dans lequel le dispositif de démêlage de tube IV est configuré pour être déplacé de manière coulissante le long de la pluralité de tubes IV (50) lorsque le boîtier (110, 210) est en position fermée, amenant les tubes IV (50) à être démêlés ou redressés.
